# EUROPEAN PATENT SPECIFICATION

(11) **EP 3 630 996 B1**
(45) Date of publication and mention of the grant of the patent: **24.03.2021**
(21) Application number: 18740669.9
(22) Date of filing: 30.05.2018
(51) Int. Cl.: C12Q 1/04, C12Q 1/06

(54) **METHOD OF USING AN IRON ADDITIVE AS A MEDIA SUPPLEMENT FOR DETECTION OF SULFATE REDUCING BACTERIA**
VERFAHREN ZUR VERWENDUNG EINES EISENADDITIVS ALS MEDIENERGÄNZUNG ZUM NACHWEIS VON SULFATREDUZIERENDEN BAKTERIEN
PROCÉDÉ D'UTILISATION D'UN ADDITIF DE FER EN TANT QUE SUPPLÉMENT DE MILIEU POUR LA DÉTECTION DE BACTÉRIES SULFATORÉDUCTRICES

(30) Priority: 02.06.2017 US 201715612358
(43) Date of publication of application: 08.04.2020
(73) Proprietor: Saudi Arabian Oil Company, Dhahran 31311 (SA)
(72) Inventor: AL-HUMAM, Abdulmohsen, A., Dhahran 31311 (SA); AL-HUMAID, Ghassan, A., Dhahran 31311 (SA)
(74) Representative: Mewburn Ellis LLP
(86) International application number: PCT/US2018/035084
(87) International publication number: WO 2018/222687

(56) References cited:
- L.A. BERNARDEZ ET AL: "Improved method for enumerating sulfate-reducing bacteria using optical density", METHODSX, vol. 2, 1 January 2015 (2015-01-01), pages 249-255, XP055497707, ISSN: 2215-0161, DOI: 10.1016/j.mex.2015.04.006
- MARÍA ISABEL SARRÓ ET AL: "Comparison Of Bacterial Growth Of Sulfate Reducing Bacteria Evaluated By Serial Dilution, Pure Plate And Epifluorescence Techniques", NACE INTERNATIONAL CORROSION CONFERENCE 2007, 1 January 2007 (2007-01-01), XP055497820,
- RANJITA BISWAS ET AL: "Improved growth rate in Clostridium thermocellum hydrogenase mutant via perturbed sulfur metabolism", BIOTECHNOLOGY FOR BIOFUELS, vol. 10, no. 1, 3 January 2017 (2017-01-03), XP055497845, DOI: 10.1186/s13068-016-0684-x
- ZHOU CHEN ET AL: "Reductive precipitation of sulfate and soluble Fe(III) byDesulfovibrio vulgaris: Electron donor regulates intracellular electron flow and nano-FeS crystallization", WATER RESEARCH, ELSEVIER, AMSTERDAM, NL, vol. 119, 19 April 2017 (2017-04-19), pages 91-101, XP085023944, ISSN: 0043-1354, DOI: 10.1016/J.WATRES.2017.04.044
- NIDAL ABU LABAN ET AL: "Anaerobic benzene degradation by Gram-positive sulfate-reducing bacteria : Anaerobic benzene degradation by Gram-positive bacteria", FEMS MICROBIOLOGY ECOLOGY., vol. 68, no. 3, 1 June 2009 (2009-06-01), pages 300-311, XP055497865, NL ISSN: 0168-6496, DOI: 10.1111/j.1574-6941.2009.00672.x
- J. LOUBINOUX ET AL: "Isolation of Sulfate-Reducing Bacteria from Human Thoracoabdominal Pus", JOURNAL OF CLINICAL MICROBIOLOGY, vol. 41, no. 3, 1 March 2003 (2003-03-01), pages 1304-1306, XP055302139, US ISSN: 0095-1137, DOI: 10.1128/JCM.41.3.1304-1306.2003

## Description

### FIELD OF THE INVENTION

The invention relates to the field of detection of structural monitoring. More specifically, the invention relates to monitoring Sulfate Reducing Bacteria (SRB) that cause degradation and corrosion damage of infrastructural metal structures used in the oil industry.

### BACKGROUND OF THE INVENTION

In the petroleum industry, colonies of microbes can grow on infrastructural equipment. In particular, the growth of sulfate-reducing bacteria (SRB) is problematic to the petroleum industry due to the ability of SRBs to reduce sulfates to sulfides. Sulfides are found to contribute to corrosion, plugging of injection wells, souring of gas and/or oil and to the increase of toxic hydrogen sulfide. These problems raise production costs significantly due to equipment failure, the additional equipment needed to remove sulfides, the remedial use of biocides to control microbial growth, and the additional chemicals employed to remove or prevent iron sulfide deposits.

Traditionally, the petroleum industry has used the Most Probable Number (MPN) method to detect the growth of SRB from field samples. The MPN method is slow and can take up to 28 days to report the final reading. Another drawback of this method is that it cannot detect low densities of viable SRB cells. Therefore, SRBs can be present in samples without being detected because their concentration is under the detection limit of the MPN method.

Direct microscopy is an alternative method that is rapid, but it does not distinguish live from dead bacteria. Other SRB test kits are also available; however, these kits have their own associated disadvantages in terms of inordinate test length times and lack of sensitivity. There is therefore a need for an SRB detection method which easily adapts to the rigors of the oil field and has enhanced sensitivity would improve detection of Sulfate Reducing Bacteria and their growth
L.A. BERNARDEZ ET AL: "Improved method for enumerating sulfate-reducing bacteria using optical density", METHODSX, vol. 2, 1 January 2015, pages 249-255 discloses an assay to determine the concentration of SRBs which grow in a medium which turns black as a consequence of the precipitation of FeS. Sequential dilutions allow for a determination of the concentration.
RANJITA BISWAS ET AL: "Improved growth rate in Clostridium thermocellum hydrogenase mutant via perturbed sulfur metabolism", BIOTECHNOLOGY FOR BIOFUELS, vol. 10, no. 1, 3 January 2017 discloses an MTC medium in to which ferrous chloride tetrahydrate has been added. It is used in several Balch tubes which qualify as vials.
ZHOU CHEN ET AL: "Reductive precipitation of sulfate and soluble Fe(III) by Desulfovibrio vulgaris: Electron donor regulates intracellular electron flow and nano-FeS crystallization", WATER RESEARCH, vol. 119, 19 April 2017, pages 91-101 and NIDAL ABU LABAN ET AL: "Anaerobic benzene degradation by Gram-positive sulfate-reducing bacteria : Anaerobic benzene degradation by Gram-positive bacteria", FEMS MICROBIOLOGY ECOLOGY., vol. 68, no. 3, 1 June 2009 (2009-06-01), pages 300-311, disclose respectively an assay medium and an enrichment for a culture medium in several vials, enriched with Ferrous chloride.

It is with respect to these and other considerations that the disclosure made herein is presented.

### SUMMARY OF THE INVENTION

According to the present invention there is provided a method of determining a concentration of sulfate reducing bacteria (SRB) as set out in claim 1. The method comprises preparing a standard growth medium in a group of test vials, adding ferrous chloride tetrahydrate (FCTH) to the each of the test vials, adding a sample portion to a first of the group of test vials, sequentially diluting the sample portion by injecting succeeding vials in the test group with fluid from sequentially previous vials and incubating the group of test vials, wherein blackening of fluid in an incubated test vial indicates a presence of a threshold number of SRB.

In some embodiments, the method of determining a concentration of SRB according to the present invention includes adding an iron source to the standard growth media in the group of test vials prior to adding the FCTH. In some embodiments, the FCTH is added at a dosage of 0.01 grams per 9 milliliters of standard growth media. In some implementation, the FCTH is added as 0.5 to 1 milliliter of solution to 8 milliliters of standard growth media and 1 ml of tested sample.

In other embodiments, the method of determining a concentration of SRB according to the present invention includes adjusting the pH of the standard growth media to compensate for addition of FCTH which is approximately 7.6. In further embodiments, the step of sequential diluting is performed by drawing one milliliter from a vial already containing mixed tested sample and injecting the drawn milliliter it to a next sequential vial. In some implementations, that group of test vials is incubated for 5 to 7 days. In some implementations, the test vials can be incubated at 37 degrees Celsius.

A test kit for determining a concentration of sulfate reducing bacteria (SRB) in a test sample is also disclosed herein. The test kit comprises a group of test vials containing a standard growth media and an iron additive comprising ferrous chloride tetrahydrate (FCTH) for adding to the test vials. During a determination, a test sample is added to a first of the group of test vials, and the test sample is sequentially diluted in remaining vials of the group. Upon incubation of the test vials with diluted sample and iron additive, a blackening of fluid in any of the test vials in the group indicates SRB concentration above a threshold level.

In some embodiments, the vials are filled with 8 milliliters of standard growth media and 0.5 to 1 milliliter of FCTH. In some implementations, the first of test vials is injected with 1 milliliter of a test sample. Additionally, the pH of the standard growth media can be adjusted to compensate for the FCTH iron additive to approximately 7.6.

These and other aspects, features, and advantages can be appreciated from the following description of certain embodiments of the invention and the accompanying drawing figures and claims.

### BRIEF DESCRIPTION OF THE DRAWINGS

FIG. 1 is a flow chart of a method for preparing a test group and a control group for determining the presence and amount of sulfate-reducing bacteria in field samples using an FCTH additive according to an embodiment of the present invention;
FIG. 2 is a flow chart of a method for applying the FCTH iron additive to the test group to prepare a test for SRB concentration according to an embodiment of the present invention;
FIG. 3 is a schematic illustration of the process of sequential dilution of a test sample with addition of FCTH according to an embodiment of the present invention; and
FIG. 4 is a schematic illustration of the results of three exemplary tests for SRB using the methods of the present invention.
FIG. 5 is a table used for calibrating dilution test results to an estimate of SRB concentration according to the prior art.

### DETAILED DESCRIPTION OF CERTAIN EMBODIMENTS OF THE INVENTION

To more quickly and accurately test for the number of SRB present in a sample, a test protocol is disclosed in which an iron compound, ferrous chloride tetrahydrate (FeSO4·7H2O) ("FCTH"), is used as an additive to a standard bacterial growth media. As described herein, this additive acts as a supplemental media to the standard media and not only increases SRB detection sensitivity but also provides for rapid detection of SRB growth. In particular, as described herein, the present method of detection of SRB relies upon a visual cue to alert the tester that SRB is present and more particularly, the presence of SRB will be clearly revealed by the growth medium having turned black due to a suspension of iron sulfides.

The protocol starts by employing a standard media, such as API RP 386, for supporting the growth of SRB and then adds the FCTH in a serially dilutive manner. Upon incubation, the FCTH boosts the production of iron sulfides by the SRB, which darken the media and provide a marker for the presence of SRB. Using vials having different degrees of dilution, the number of SRB present can be estimated according to the degree of dilution at which iron sulfide darkening is visually detected in the sample (i.e., the growth medium turns black). The addition of FCTH improves the lower limit of detection to approximately 10⁴ to 10⁵ cells per milliliter.

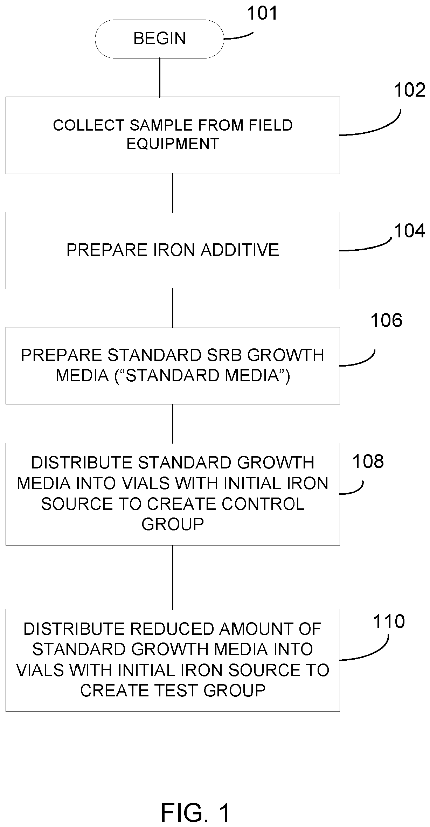

FIG. 1 is a flow chart of an embodiment of a method for preparing a test group and a control group for determining the presence and amount of sulfate-reducing bacteria in field samples according to the present invention. In step 101, the method begins. In step 102, samples are collected from field equipment (e.g., oil pipelines, well holes, storage vessels) to determine whether the samples contain SRB, and if so, the amount of SRB that they contain. In a second step 104, an iron additive is prepared. In some implementations, this step involves dissolving 1 gram of FCTH in 100 ml of distilled water, purging the solution with argon gas, and then sealing and autoclaving the solution for approximately 15 minutes (which yields an iron additive solution of concentration 10 mg/ml).

In step 106, a standard SRB growth media ("standard media") is prepared. In some implementations, the standard media is prepared using water and the components shown below in Table 1.

**TABLE 1**

| COMPONENT | Weight (g/1.0L) |
|---|---|
| Ammonium Chloride (NH₄Cl) | 1 |
| Calcium Sulfate (CaSO₄.2H₂O) | 1 |
| Magnesium Sulfate (MgSO₄.7H₂O) | 2 |
| Sodium Lactate (C₃H₅O₃Na) | 3.5 mL |
| Yeast Extract | 1 |
| Ascorbic Acid | 0.1 |
| Resazurin solution (25 mg/100 H₂O) | 4.0mL |
| Sodium Thioglycollate (CH₂SHCOONa) | 0.1 |
| Ferrous Sulfate (FeSO₄.7H₂O) | 0.5 |
| Potassium Hydrogen Phosphate (K₂HPO₄) | 0.5 |

During preparation of the standard media, the salinity of the water used to create the media can be adjusted to match the salinity of the samples (e.g., water (synthetic or filtered original water) with similar salinity can be used). In addition, the pH of the standard media can be adjusted for the presence of iron. For example, the pH of the prepared media without an iron supplement can be compared to the pH of the standard media including a supplement of 1 ml of iron. If the pH of the two media is significantly different, the pH of the standard media that includes the iron supplement can be adjusted to match the pH of the standard media without the iron supplement. In general, the components listed in Table 1 can be prepared separately to avoid precipitation and to maintain anaerobic conditions.

After the standard media has been prepared and the salinity adjusted, in step 108, a test control group is created by distributing a quantity of the standard media into a group of vials, into each of which an iron source, such as an iron nail, is also added. The vials are then sealed. In a particular implementation, 9 ml of the standard media is distributed to eight or more vials. In step 110, a test group is prepared similarly by distributing a smaller amount of standard media into a second group of vials ("test group"), into each of which an iron source, such as a nail, as well as FCTH is also added. The test group vials are then sealed as well. In a particular implementation, 8 ml of standard media is distrusted to the vials of the test group. Afterwards 0.5 ml of stack solution including FCTH (1 g/ml of solution) is then added to test group.

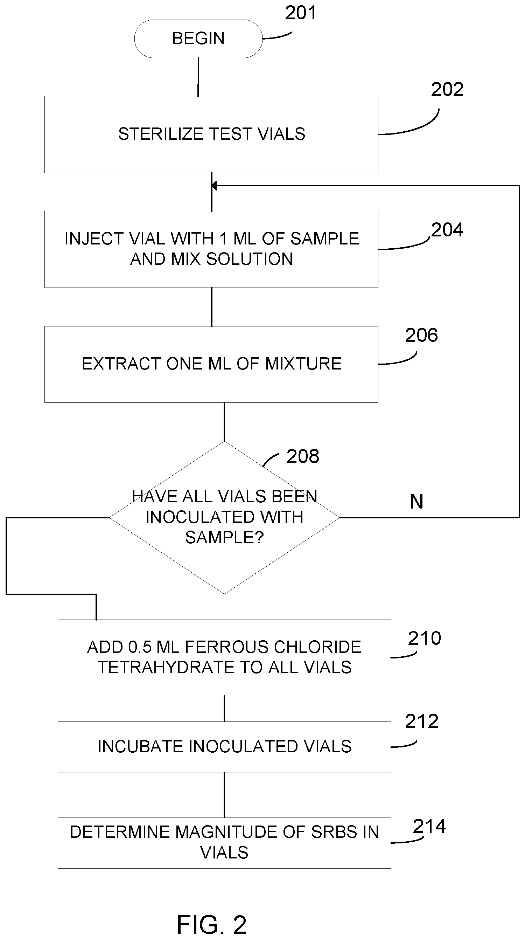

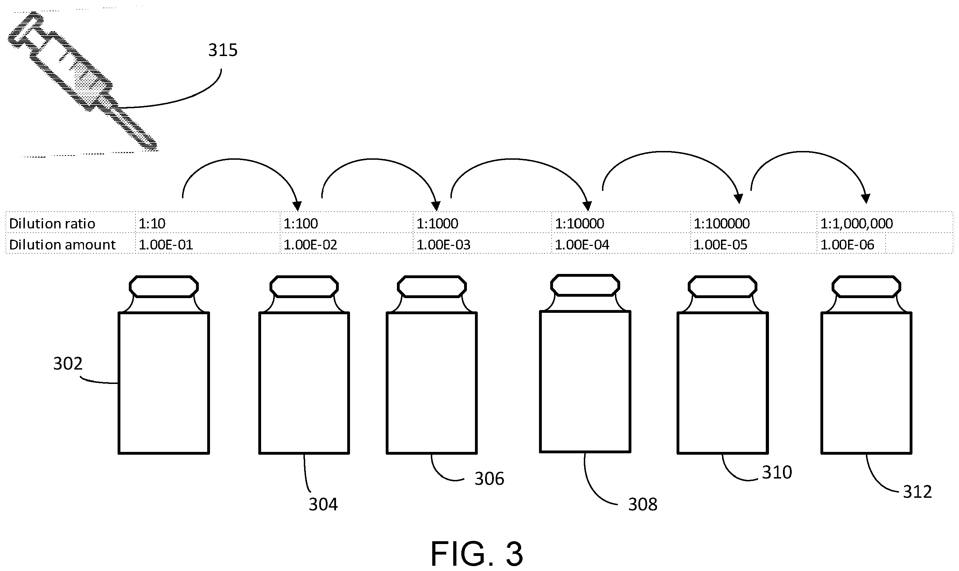

FIG. 2 is a flow chart of a method for applying the FCTH iron additive to the test group to prepare a test for SRB concentration. In step 201, the method begins. In step 202, the test vials are sterilized for one hour in an autoclave or similar sterilization device. After sterilization, in step 204 the first vial of the test group is injected with 1.0 ml of the sample to be tested and the solution is then mixed thoroughly. In step 206, one ml of the mixture is extracted from the first vial which is then added to the next vial of the test group. In this manner the successive vial receives a diluted portion (i.e., one tenth concentration) of the sample. The process of sequential addition and extraction is repeated until it is determined in step 208 that all eight or more of the vials in the test group have been inoculated with a portion of sample. This process is schematically illustrated in FIG. 3. As shown, a test group includes six vials 302, 304, 306, 308, 310 and 312. Although six vials are shown, this is merely for ease of illustration, and a smaller, or preferably larger, number of vials can be used. A syringe 315 is shown which is able to both inject fluid into and draw fluid out of the vials 302-312. The first vial 302 includes 9 ml of fluid and is injected with one ml of sample, yielding a 1:10 sample/fluid ratio, or one tenth of the original sample concentration. When 1 ml of the fluid is drawn from vial 302 and injected into vial 304, vial 304 then effectively contains one-tenth of one-tenth of the original sample concentration, or a 1:100 sample/fluid ratio. During the process of sequentially drawing 1 ml of fluid from a vial and injecting the drawn fluid into the next sequential vial, the sample/fluid ratio decreases geometrically: vial 306 has a 1:1000 ratio, vial 308 has a 1:10,000, vial 310 has a 1:100,000 ratio, and vial 312 has a 1:1,000,000 ratio.

After inoculation of the test group, in step 210, an amount of FCTH, such as 0.5 ml, is added (e.g., by injection) to all of the vials (that contain the diluted sample) and the resulting solution is thoroughly mixed. In step 212, the sequentially diluted test group vials are incubated for a period at 37 degrees Celsius. The preferred period of incubation is five (5) to seven (7) days, a reduction of at least 75 percent over the prevailing method which requires 28 days. After incubation, in step 214 the presence of SRB is revealed, and their magnitude is determined, when the medium in the test group vials turns black due to a suspension of iron sulfides.

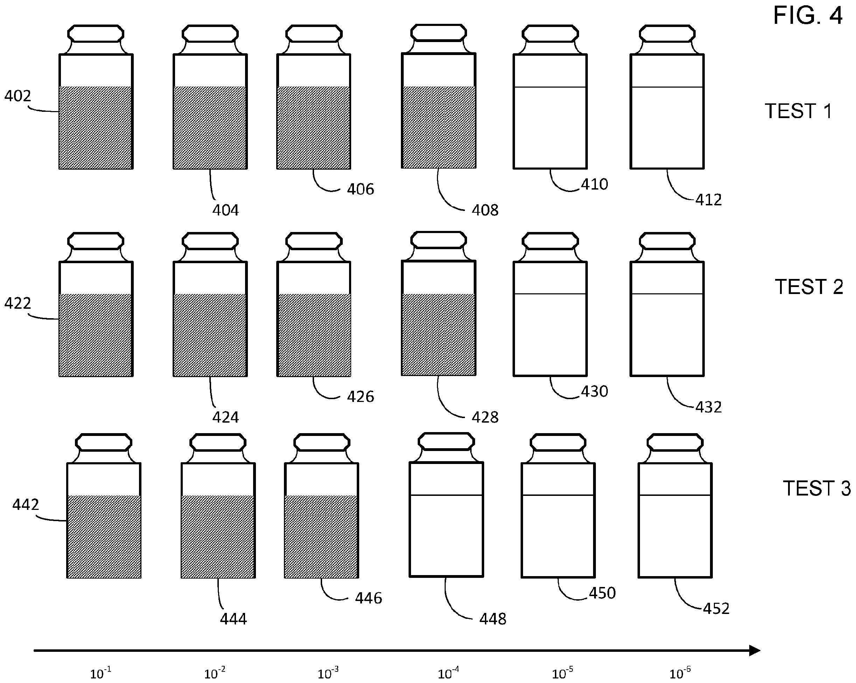

FIG. 4 is a schematic illustration showing three exemplary sample tests, test 1, test 2 and test 3 of different original samples. As shown, in test 1 the fluid in four vials 402, 404, 406 and 408 is shown as having blackened, indicating a threshold concentration of SRB, (while the fluid in two vials 410, 412 is shown as not having blackened, indicating that the SRB concentration is lower than the threshold in vials 410, 412. Vials 402, 404, 406 and 408 represent the 10⁻¹, 10⁻², 10⁻³ and 10⁻⁴ sample/fluid ratios, respectively, while vials 410 and 412 represent the 10⁻⁵ and 10⁻⁶ sample/fluid ratios, respectively. This shows that beyond the 1:10,000 dilution level of the original sample, there is insufficient SRB concentration to cause blackening of the fluid by production of iron sulfide. Similarly, in test 2, the fluid in four vials 422, 424, 426 and 428 is shown as having blackened, again indicating a threshold concentration of SRB, while the fluid in two vials 430, 432 is shown as not having blackened, indicating that the SRB concentration is lower than a threshold in vials 430, 432. Vials 422, 424, 426 and 428 represent the 10⁻¹, 10⁻², 10⁻³ and 10⁻⁴ sample/fluid ratios, respectively, while vials 430 and 432 represent the 10⁻⁵ and 10⁻⁶ sample/fluid ratios, respectively. Test 2 similarly shows that beyond the 1:10,000 dilution level of the original, there is insufficient SRB concentration to cause blackening of the fluid by production of iron sulfide. Thus, in tests 1 and 2, the blackening of the fluid at the 10⁻⁴ dilution level gives an indication of the magnitude of SRB in the original samples used in the tests, which can be converted into a measurement of the concentration of SRB present in the original samples used in test 1 and test 2.

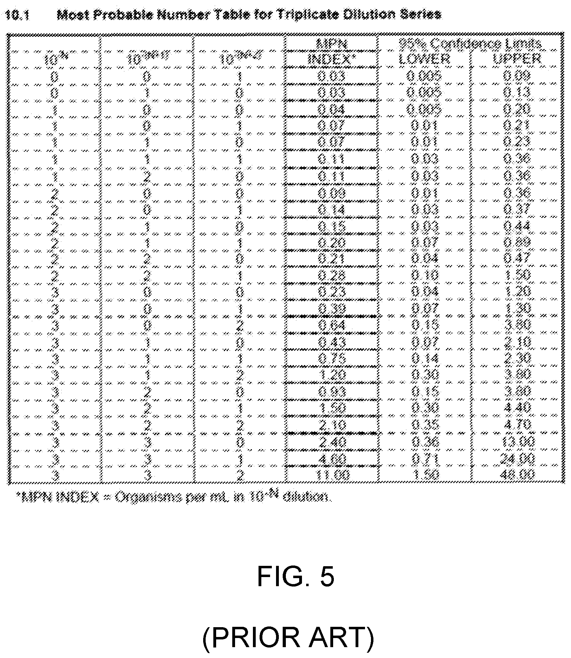

The dilution level at which blackening occurs can be converted with a sufficient degree of accuracy to an estimate of the number of SRB per ml in a sample using the most probable number (MPN) calibration method. The MPN calibration method utilizes a table in which results of standard dilution tests are calibrated with an estimate of organism concentration. As an example, The following example illustrates how this method is used. A sample is taken and used in three serial dilution tests for SRB concentration. The vial results are shown in Table 2 directly below in which "+" indicates blackening and "-" indicates no blackening. From the test results, the fifth, sixth and seventh dilutions are selected to provide an estimate of the concentration of SRB in the sample. As indicated in Table 2, all three tests were positive at the 10⁻⁵ level (10^{-N} in the MPN table of FIG. 5), 2 tests were positive at the 10⁻⁶ level (10^{-N+1} in the MPN table) and 1 test was positive at the 10⁻⁷ level (10^{-N+2} in the MPN table). Matching this 3-2-1 pattern in the MPN table to an MPN estimate gives a value of 1.5. Multiplication by the dilution factor yields a value of 1.5 x 10⁵ SRB in the original sample.

**Table 2: Example calibration with MPN table**

| First dilution | Second dilution | Third dilution | Fourth dilution | Fifth dilution | Sixth dilution | Seventh dilution | Eighth dilution |
|---|---|---|---|---|---|---|---|
| 10⁻¹ | 10⁻² | 10⁻³ | 10⁻⁴ | 10⁻⁵ | 10⁻⁶ | 10⁻⁷ | 10⁻⁸ |
| + | + | + | + | + | - | - | - |
| + | + | + | + | + | + | + | -- |
| + | + | + | + | + | + | - | - |

In contrast, in test 3 the fluid in only three of the vials 442, 444, 446 have blackened, indicating a threshold concentration of SRB, while the fluid in two vials 448, 450 and 452 is shown as not having blackened, indicating that the SRB concentration is lower than the threshold in vials 448, 450 and 452. Vials 442, 444, and 446 represent the 10⁻¹, 10⁻² and 10⁻³ sample/fluid ratios, respectively, while vials 448, 450 and 452 represent the 10⁻⁴, 10⁻⁵ and 10⁻⁶ sample/fluid ratios, respectively. Thus, test 3 differs from tests 1 and 2 in that beyond the 1:1,000 dilution level of the original (as opposed to the 1:10,000 level), there is insufficient SRB concentration to cause blackening of the fluid by production of iron sulfide.

A number of tests were conducted in which various types of water samples with different salinity and SRB cultures were used. SRB cultures were prepared and isolated from different locations, namely, the Abu Ali water supply, Abu Ali disposal water, hard water supply and Uthmaniyah water supply. Different volumes (i.e., 0.1ml and 0.2 ml) of SRB culture were injected in 100 ml of distilled water. After that, 1 ml from the total volume was used as inoculum to conduct the MPN test. This inoculum was conducted with different water salinity. Tables 1-6 set forth different test examples and more specifically: Example 1: SRB culture (35% of QSW) - a) 0.1 ml SRB/100 distilled water; and b) 0.2 ml SRB/100 distilled water; Example 2: SRB culture (100% of QSW) - a) 0.1 ml SRB/ 100 distilled water; and b) 0.2 ml SRB/100 distilled water; Example 3: Abu Ali water supply well (35% of QSW); Example 4: Abu Ali disposal water (100% of QSW); Example 5: hard water sample (100% of QSW); and Example 6: Uthmaniyah water samples (100% of QSW).

The experiments were conducted under the following conditions: for each test sample, two triplicate 16-dilution series of MPN were tested. One MPN set used the standard SRB media (9 ml of media in each vial), while the other MPN set was prepared by adding 1 ml of iron additive to the standard media resulting in 9 ml of media in each vial after addition of the iron additive. A water sample of 1 ml was inoculated for the 16 dilution series in triplicate. The samples were incubated for 28 days at 35°C. Growth was checked daily and the day in which the sample (culture) turned black was recorded in order to confirm whether the iron additive (IC) expedites SRB growth. After 28 days, the final MPN reading and data conversion was conducted and this indicates whether the iron additive improves SRB detection sensitivity.

Table 2, set forth below, shows SRB growth that results using the standard MPN method (described herein) with and without the addition of 0.1 ml and 0.5 ml of iron compound at the 0.1 ml SRB culture in 35% of Qurayyah seawater (QSW). The below data indicates that in the absence of the iron compound (IC) which is represented by the original sample column, low growth of 2.3 SRB/ml was detected after one day (i.e., day 1) of incubation. After two days, SRB growth increased to 230 SRB/ml but then remained at this level up to the end of the incubation period of 28 days. However, with the addition of 0.1 ml of iron compound (IC), the results show that 2.3 SRB/ml was detected after one day (day 1) of incubation. After two days, SRB growth increased to 2.3X10⁴ SRB/ml and remained at this level up to the end of the incubation period of 28 days. Similar results were detected with the addition of 0.5 ml of iron compound (IC). The results thus show that 2.3 SRB/ml was detected after one day of incubation. After two days, SRB growth was increased to 2.3X10⁵SRB/ml and remained up to the end of the incubation period of 28 days. The test results thus indicate that the iron compound (IC) not only expedites SRB growth but also improves SRB detection sensitivity.

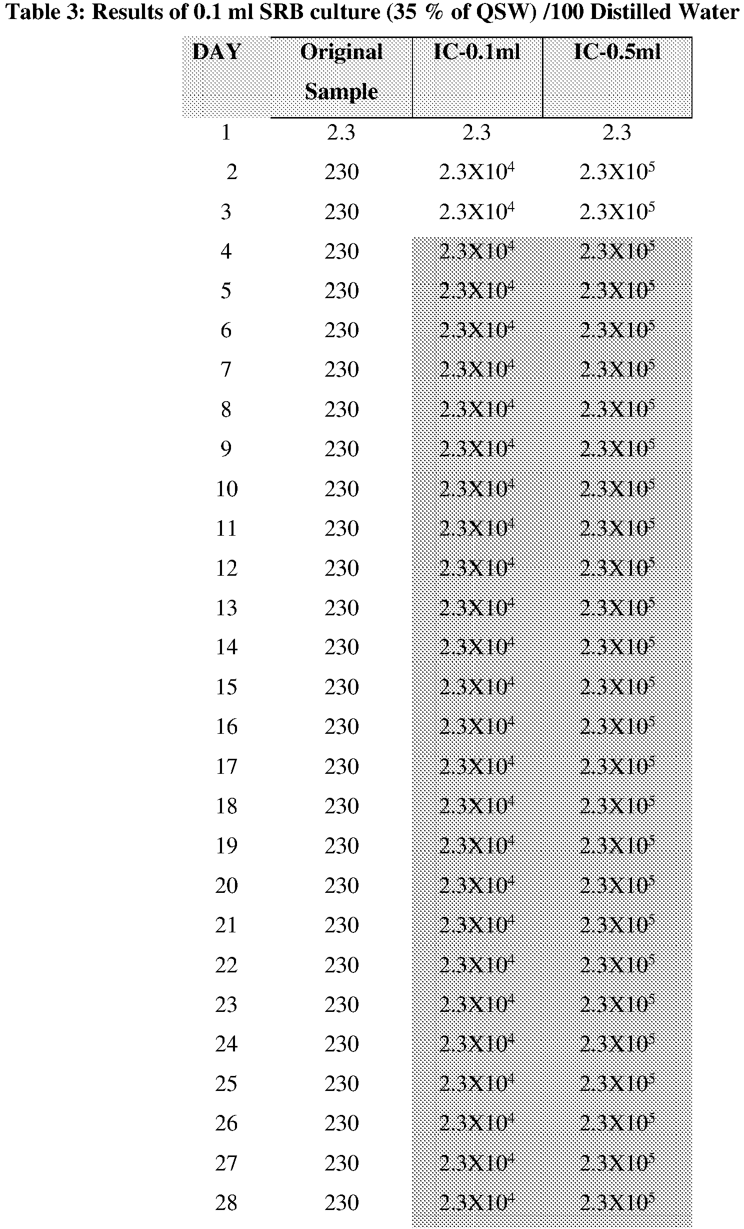

Table 3 shows the SRB growth results with and without the addition of 0.1 ml and 0.5 ml of iron compound to the 0.2 ml SRB culture in 35% of Qurayyah seawater (QSW). The data indicates that in the absence of the iron compound low growth of 2.3 SRB/ml was detected after one day of incubation. After two days, SRB growth was increased to 230 SRB/ml and remained at this level up to the end of the incubation period of 28 days. However, with the addition of 0.1 ml of iron compound, the result show that 2.3 SRB/ml was detected after one day of incubation. After two days, SRB growth was increased to 2.3X10³ SRB/ml and remained up to the end of the incubation period of 28 days. Comparable results were detected with the addition of 0.5 ml of iron compound. The results show that 230 SRB/ml was detected after one day incubation. Later, SRB growth was increased to 2.3X10⁴ SRB/ml and remained up to the end of the incubation period of 28 days. The test results thus indicate that the iron compound (IC) not only expedites SRB growth but also improves SRB detection sensitivity.

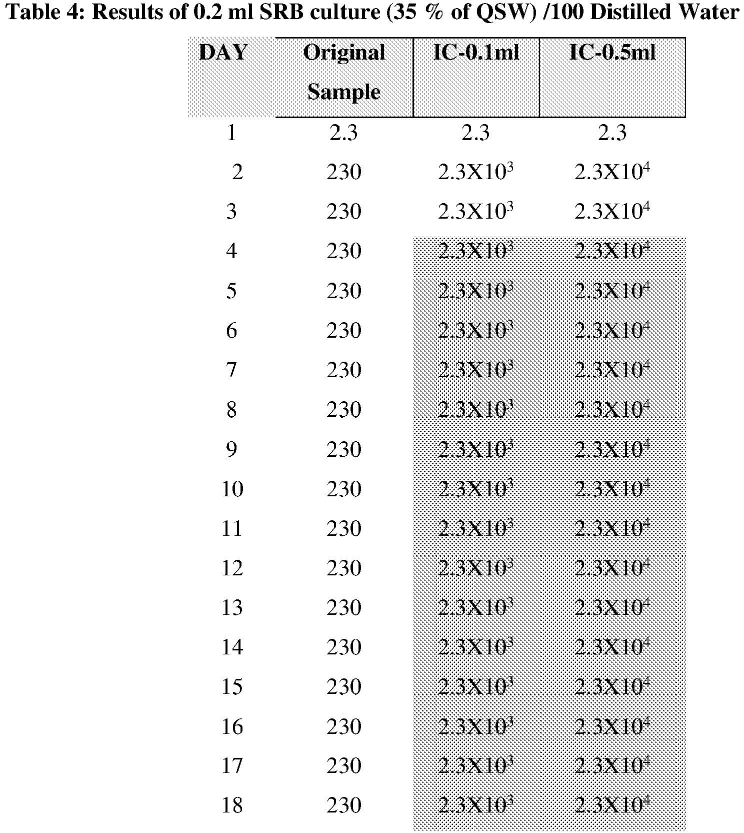

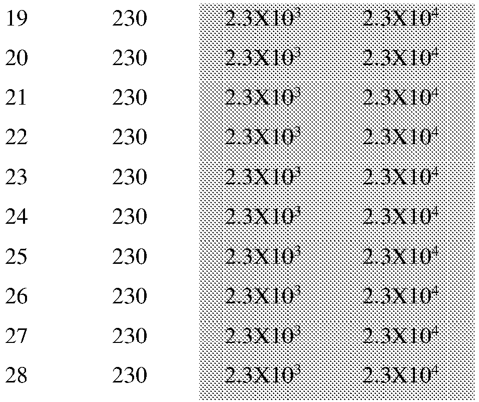

Table 4 shows SRB growth results with and without the addition of 0.5ml of iron compound to the 0.1 ml SRB culture in 100% of Qurayyah seawater (QSW). The result shows that in the absence of the iron compound, low growth of 2.3 SRB/ml was detected after one day of incubation. After two days, SRB growth was increased to 2.3X10² SRB/ml. Later, SRB growth was increased to 2.3X10⁴ SRB/ml in the third day. Subsequently, SRB growth continued to increase significantly to 2.3X10¹² SRB/ml in the fifth day and remained at this level to the end of the incubation period. Similar results were detected with the addition of 0.5 ml of iron compound. SRB growth was detected at 2.3 SRB/ml after one day of incubation. Two days later, SRB growth increased to 2.3X10² SRB/ml then to 2.3X10⁴ SRB/ml after two days, respectively. On the fifth day, the SRB growth increased to 2.3X10¹⁷ SRB/ml, and remained at this level to the end of the incubation period of 28 days. The test results thus indicate that the iron compound (IC) not only expedites SRB growth but also improves SRB detection sensitivity.

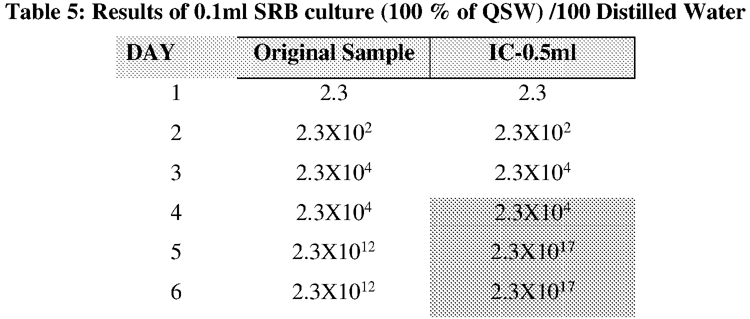

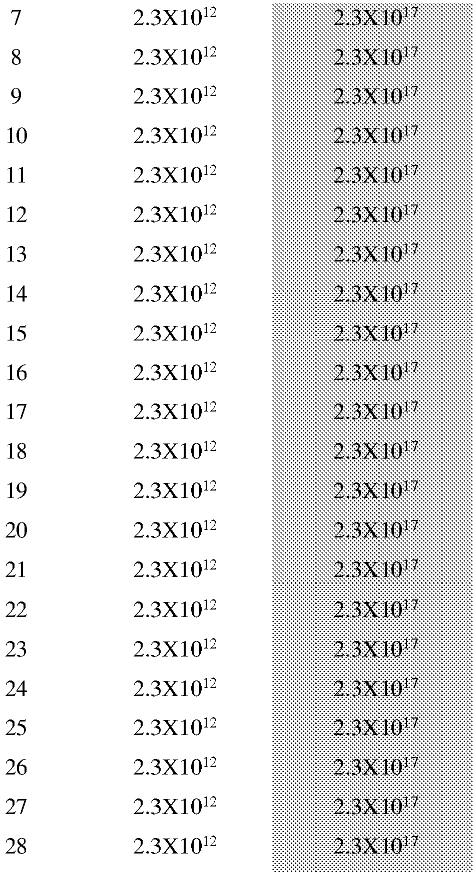

The results in Table 5 shows SRB growth results with and without the addition of 0.5ml of iron compound to the 0.2 ml SRB culture in 100% of Qurayyah seawater (QSW). The results indicate that in the original sample without iron compound addition, the SRB growth was below the detection limit, for the first two days of incubation. A very slight increase to 23 SRB/ml was detected on the fourth day, and continued at this level to the end of the incubation period. Identical results were detected with the addition of 0.1 ml of the iron compound. SRB growth was below the detection limit, for the first two days of incubation. But, very minor increase to 23 SRB/ml was detected on the fourth day, and continued at this level to the end of the incubation period. However, with the addition of 0.5 ml of Iron compound, SRB growth was below the detection limit after one day of incubation. But after two days, the result show that the SRB growth was slightly increased to 2.3 SRB/ml. After that, SRB growth was increased to 2.3X102 SRB/ml in the third day, and again increased to 2.3X104 SRB/ml after four days, and stayed at this level up to the end of the incubation period of 28 days. The test results thus indicate that the iron compound (IC) not only expedites SRB growth but also improves SRB detection sensitivity.

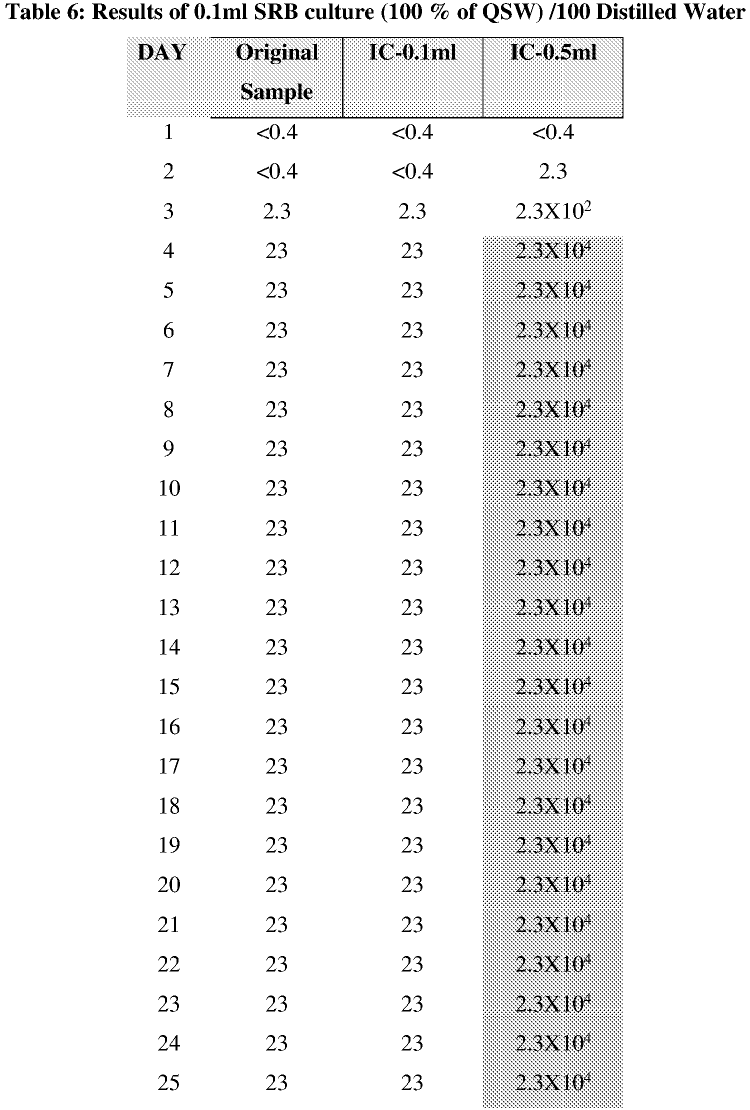

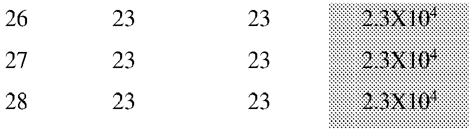

SRB results for Abu Ali water supply well- 840 (35% of QSW) are shown in Table 6. The test was designed to detect SRB growth without and with the addition of 0.5ml of iron compound at the 0.5 ml. The result indicated that in original sample without iron compound addition, SRB growth was below the detection limit, during the 28 days of incubation period. Identical result was detected with the addition of 0.5 ml of the iron compound. SRB growth was below the detection limit up to the fifth day. However, the SRB growth was increased gradually to 2.3 SRB/ml, then to 2.3X10² SRB/ml after five and ten days, respectively, and stayed at this level up to the end of the incubation period of 28 days. The test results thus indicate that the iron compound (IC) not only expedites SRB growth but also improves SRB detection sensitivity.

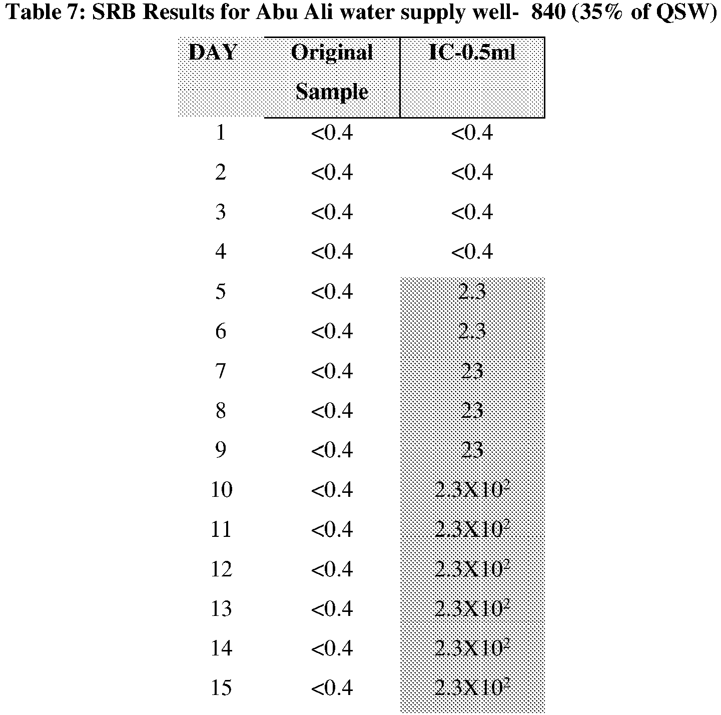

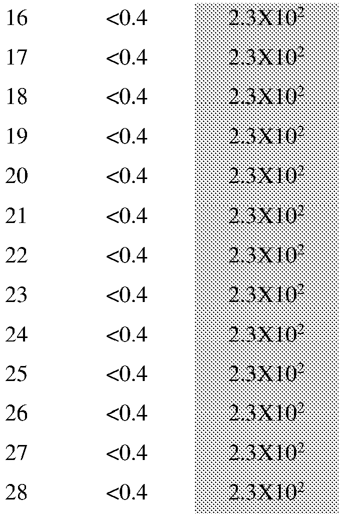

SRB results for a hard water sample are presented in Table 7. The result shows that in the absence of the iron compound addition (original sample), the SRB growth was below the detection limit during the 28 days. In addition, identical results were detected with the addition of 0.2 ml of iron compound. SRB growth was below the detection limit for 28 days. However, with the addition of 0.5 ml of iron compound, SRB growth was below detection limit for the first five days. But after six days, SRB growth was slightly increased to 2.3 SRB/ml, and remained at this level to the end of the incubation period of 28 days.

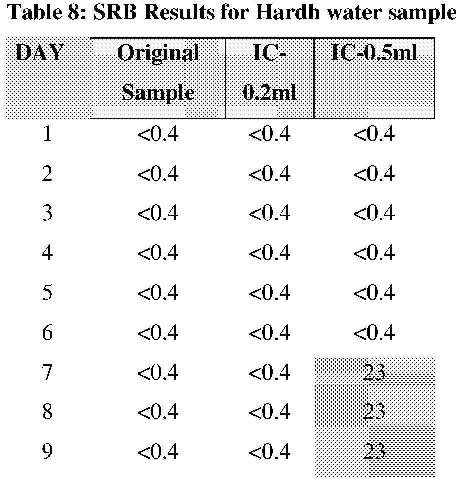

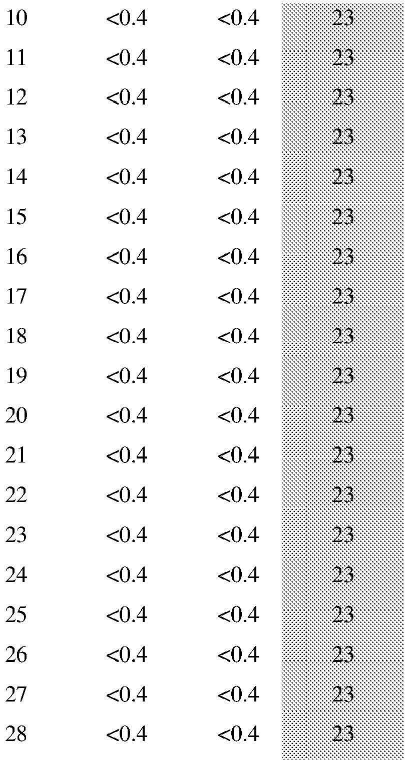

Table 8 shows the SRB results for hard water sample, with and without the addition of 0.2ml and 0.5ml of iron compound. The results show that in the absence of the iron compound addition, SRB growth was below the detection limit during the all incubation period. Identical result was detected with the addition of 0.2 ml of iron compound. SRB growth was below the detection limit for 28 days. However, with the addition of 0.5 ml of iron compound, SRB growth rate was below the detection limit for the first two days. But after four days, the SRB growth was slightly increased to 2.3 SRB/ml, and remained at this level to the end of the incubation period of 28 days.

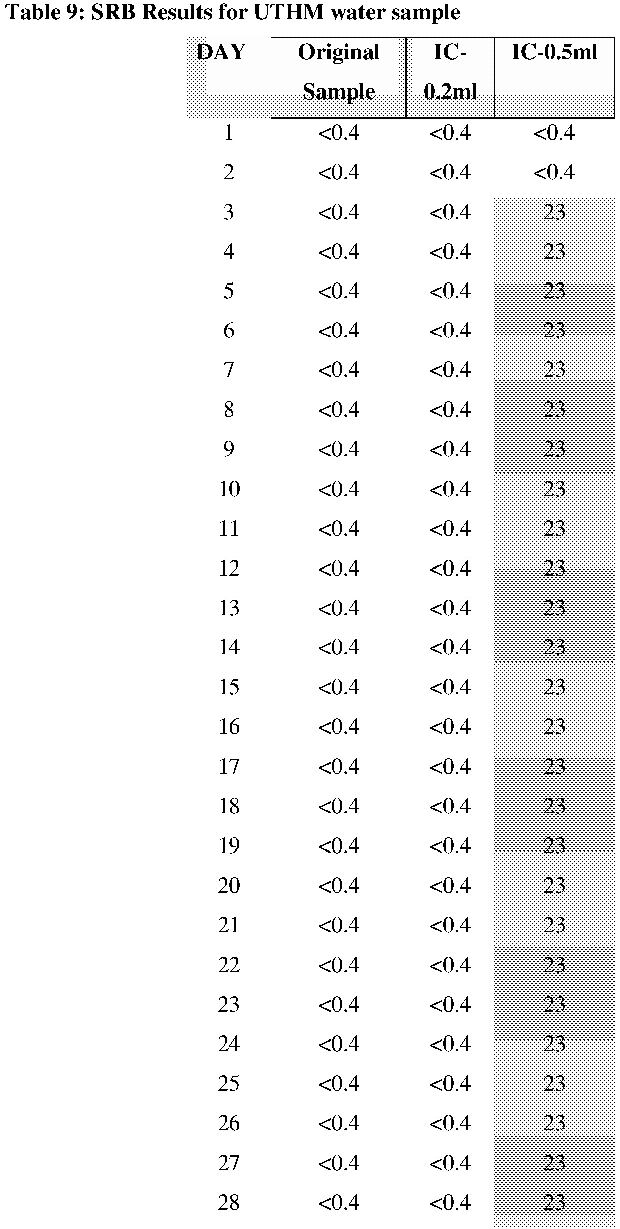

The methods of determining SRB concentration according to the present invention provide several advantages over the current art. The methods enable detection of very low concentrations of viable SRB cells, typically below the detection limit of any known bacterial growth culture. Furthermore, the methods provide for the rapid re-activation of dormant SRB, particularly SRB that cannot be regrown using standard procedures and are typically wrongly perceived to be no longer alive. Significantly, the methods enable rapid growth an detection of SRB, generally within 5-7 days, which is at least 75% less time than required for the growth of SRB from oilfield samples using standard media (i.e., up to 28 days).

Applicant has discovered that the additional of the FCTH enhances and boosts the activity of SRBs and activates dormant SRBs as supported by the present disclosure and the examples contained herein.

As discussed herein, the present invention is directed to a media supplement in the form of an iron compound (IC) is ferrous chloride tetra-hydrate (FeCl2.4H2O) that was added to SRB standard media that is used for most probable number-MPN method. The following observations were made:
1. Iron compound acts as a supplement media to increase the SRB detection sensitivity and promote the detection of SRB growth.
2. Rapid growth and detection of SRB (usually within 5-7 days) after the addition of iron compound.
3. In the absence of the iron compound, the MPN method cannot detect very low densities of viable SRB cells.
4. In the absence of the iron compound, the MPN method very slow re-activation of the dormant SRB.
5. The addition of the iron compound with different volume were effective. However, 0.5 ml from the IC stock Iron salutation (1 g FeCl₂.4H₂O in 100 ml distilled water) was one preferred effective volume.
6. The amount of IC will can range from 50 to 55 mg/L.

The rapid detection of SRB provided by the methods of the present invention can be applied in design of microbial control kits, tools and treatment programs (e.g., biocide treatment). The methods enable rapid evaluation and adjustments of treatments, maximizing SRB control and reducing operating costs significantly. Moreover, the methods of the present invention help in minimizing the environmental impact from application of chemicals used in the procedures, and aid in optimizing such application.

It is to be understood that any structural and functional details disclosed herein are not to be interpreted as limiting the systems and methods, but rather are provided as a representative embodiment and/or arrangement for teaching one skilled in the art one or more ways to implement the methods.

It is to be further understood that like numerals in the drawings represent like elements through the several figures, and that not all components and/or steps described and illustrated with reference to the figures are required for all embodiments or arrangements.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising", when used in this specification, specify the presence of stated features, integers, steps, operations, elements, and/or components, but do not preclude the presence or addition of one or more other features, integers, steps, operations, elements, components, and/or groups thereof.

The method steps discussed above can be performed in different orders without departing from the scope of the invention. For instance, the iron additive can be prepared after preparation of the standard growth medium.

Also, the phraseology and terminology used herein is for the purpose of description and should not be regarded as limiting. The use of "including," "comprising," or "having," "containing," "involving," and variations thereof herein, is meant to encompass the items listed thereafter and equivalents thereof as well as additional items.

**I**t is intended that the invention not be limited to the particular embodiment disclosed as the best mode contemplated for carrying out this invention, but that the invention will include all embodiments falling within the scope of the appended claims.

## Claims

1. A method of determining a concentration of sulfate reducing bacteria (SRB) comprising:
preparing a growth medium for growing SRB in a group of test vials;
adding ferrous chloride tetrahydrate (FCTH) to the each of the test vials (302 to 312; 402 to 452);
adding a tested sample to a first of the group of test vials (402 to 412);
sequentially diluting the tested sample by injecting succeeding vials (422 to 452) in the group with fluid from sequentially previous vials (402 to 412); and
incubating the group of test vials (302 to 312; 402 to 452);
wherein visual blackening of fluid in one incubated test vial (302 to 312; 402 to 452) indicates a presence of a threshold number of SRB.

2. The method of claim 1, further comprising:
adding an iron source to the growth media in the group of test vials (302 to 312; 402 to 452) prior to adding the FCTH.

3. The method of claim 1, wherein the FCTH is added at a dosage of 0.01 grams per 9 milliliters of growth media.

4. The method of claim 3, wherein the FCTH is added as 0.5 to 1 milliliter of solution to 8 milliliters of growth media and 1 ml of tested sample.

5. The method of claim 1, further comprising:
adjusting the pH of the growth media to a pH of approximately 7.6 to compensate for addition of FCTH.

6. The method of claim 1, wherein the step of sequential diluting is performed by drawing one milliliter from a vial (302 to 312; 402 to 452) already containing mixed tested sample and injecting the drawn milliliter into a next sequential vial (302 to 312; 402 to 452).

7. The method of claim 1, wherein the group of test vials (302 to 312; 402 to 452) is incubated for 5 to 7 days.

8. The method of claim 7, wherein the group of test vials (302 to 312; 402 to 452) is incubated at 37 degrees Celsius.

9. The method of claim 1, wherein the number of SRB present in each test vial (302 to 312; 402 to 452) can be estimated according to the degree of dilution at which visual blackening, which indicates presence of iron sulfides, is visually detected in the sample as a result of the test vials having different degrees of dilution.

10. The method of claim 1, further including the step of estimating an SRB concentration using the most probable number (MPN) method.

11. The method of claim 1, wherein the FCTH comprises a stock iron solution that is made by adding 1 g FeCl₂.4H₂O in 100 ml distilled water and 0.5 ml of the stock iron solution is added to 8 milliliters of the growth media and 1 ml of tested sample.

12. The method of claim 1, wherein the growth media comprises an aqueous solution that includes the following components:
| COMPONENT | Weight (g/1.0L) |
|---|---|
| Ammonium Chloride (NH₄Cl) | 1 |
| Calcium Sulfate (CaSO₄.2H₂O) | 1 |
| Magnesium Sulfate (MgSO₄.7H₂O) | 2 |
| Sodium Lactate (C₃H₅O₃Na) | 3.5 mL |
| Yeast Extract | 1 |
| Ascorbic Acid | 0.1 |
| Resazurin solution (25 mg/100 H₂O) | 4.0mL |
| Sodium Thioglycollate (CH₂SHCOONa) | 0.1 |
| Ferrous Sulfate (FeSO₄.7H₂O) | 0.5 |
| Potassium Hydrogen Phosphate (K₂HPO₄) | 0.5 |

## Patentansprüche

1. Verfahren zum Bestimmen der Konzentration von sulfatreduzierenden Bakterien (SRB), das Folgendes umfasst:
das Herstellen eines Nährmediums zum Züchten von SRB in einer Gruppe von Testphiolen;
das Zusetzen von Eisen(II)-chlorid-tetrahydrat (FCTH) zu jeder der Testphiolen (302 bis 312; 402 bis 452);
das Zusetzen einer Testprobe in eine erste aus der Gruppe von Testphiolen (402 bis 412);
das aufeinanderfolgende Verdünnen der Testprobe durch Injizieren von Flüssigkeit aus aufeinanderfolgenden vorangegangenen Phiolen (402 bis 412) in darauf folgende Phiolen (422 bis 452) in der Gruppe; und
das Inkubieren der Gruppe von Testphiolen (302 bis 312; 402 bis 452);
wobei eine sichtbare Schwarzfärbung der Flüssigkeit in einer inkubierten Testphiole (302 bis 312; 402 bis 452) das Vorliegen eines SRB-Schwellenwerts anzeigt.

2. Verfahren nach Anspruch 1, das außerdem Folgendes umfasst:
das Zusetzen einer Eisenquelle zu dem Nährmedium in der Gruppe von Testphiolen (302 bis 312; 402 bis 452) vor dem Zusetzen von FCTH.

3. Verfahren nach Anspruch 1, wobei das FCTH in einer Dosierung von 0,01 g pro 9 ml Nährmedium zugesetzt wird.

4. Verfahren nach Anspruch 3, wobei das FCTH in Form von 0,5 bis 1 ml einer Lösung zu 8 ml Nährmedium und 1 ml Testprobe zugesetzt wird.

5. Verfahren nach Anspruch 1, das außerdem Folgendes umfasst:
das Einstellen des pH des Nährmediums auf einen pH von etwa 7,6 um den Zusatz von FCTH auszugleichen.

6. Verfahren nach Anspruch 1, wobei der Schritt des aufeinanderfolgenden Verdünnens durch Entnehmen von einem Milliliter aus einer Phiole (302 bis 312; 402 bis 452), die eine bereits gemischte Testprobe enthält, und Injizieren des entnommenen Milliliters in die nächste der nachfolgenden Phiolen (302 bis 312; 402 bis 452) durchgeführt wird.

7. Verfahren nach Anspruch 1, wobei die Gruppe von Testphiolen (302 bis 312; 402 bis 452) 5 bis 7 Tage lang inkubiert wird.

8. Verfahren nach Anspruch 7, wobei die Gruppe von Testphiolen (302 bis 312; 402 bis 452) bei 37 °C inkubiert wird.

9. Verfahren nach Anspruch 1, wobei die Anzahl an der in jeder Testphiole (302 bis 312; 402 bis 452) enthaltenen SRB aufgrund dessen, dass die Testphiolen unterschiedliche Verdünnungsgrade aufweisen, anhand des Verdünnungsgrads beurteilt werden kann, bei dem in der Probe eine sichtbare Schwarzfärbung, die die Gegenwart von Eisensulfiden anzeigt, visuell detektiert wird.

10. Verfahren nach Anspruch 1, das außerdem den Schritt des Beurteilens der SRB-Konzentration unter Anwendung des Verfahrens der wahrscheinlichsten Anzahl (MPN) umfasst.

11. Verfahren nach Anspruch 1, wobei das FCTH eine Eisen-Stammlösung umfasst, die durch Zusetzen von 1 g FeCl₂.4H₂O zu 100 ml destilliertem Wasser hergestellt wird, wobei 0,5 ml der Eisen-Stammlösung zu 8 ml Nährmedium und 1 ml Testprobe zugesetzt werden.

12. Verfahren nach Anspruch 1, wobei das Nährmedium eine wässrige Lösung umfasst, die die folgenden Komponenten umfasst:
| KOMPONENTE | Gewicht (g/1,0 l) |
|---|---|
| Ammoniumchlorid (NH₄Cl) | 1 |
| Calciumsulfat (CaSO₄.2H₂O) | 1 |
| Magnesiumsulfat (MgSO₄.7H₂O) | 2 |
| Natriumlactat (C₃H₅O₃Na) | 3,5 ml |
| Hefeextrakt | 1 |
| Ascorbinsäure | 0,1 |
| Resazurin-Lösung (25 mg/100 H₂O) | 4,0 ml |
| Natriumthioglykolat (CH₂SHCOONa) | 0,1 |
| Eisen(II)-sulfat (FeSO₄.7H₂O) | 0,5 |
| Kaliumhydrogenphosphat (K₂HPO₄) | 0,5 |

## Revendications

1. Procédé de détermination d'une concentration de bactéries réductrices de sulfate (SRB), comprenant les étapes consistant à :
préparer un milieu de croissance pour faire croître des SRB dans un groupe de flacons de test ;
ajouter du tétrahydrate de chlorure ferreux (FCTH) dans chacun des flacons de test (302 à 312 ; 402 à 452) ;
ajouter un échantillon testé à un premier du groupe de flacons de test (402 à 412) ;
diluer séquentiellement l'échantillon testé par injection de flacons successifs (422 à 452) dans le groupe avec du fluide provenant de flacons séquentiellement précédents (402 à 412) ; et
incuber le groupe de flacons de test (302 à 312 ; 402 à 452) ;
dans lequel un noircissement visuel de fluide dans un flacon de test incubé (302 à 312 ; 402 à 452) indique la présence d'un nombre seuil de SRB.

2. Procédé selon la revendication 1, comprenant en outre l'étape consistant à :
ajouter une source de fer aux milieux de croissance dans le groupe de flacons de test (302 à 312 ; 402 à 452) avant d'ajouter le FCTH.

3. Procédé selon la revendication 1, dans lequel le FCTH est ajouté à un dosage de 0,01 gramme pour 9 millilitres de milieu de croissance.

4. Procédé selon la revendication 3, dans lequel le FCTH est ajouté sous la forme de 0,5 à 1 millilitre de solution à 8 millilitres de milieux de croissance et 1 ml d'échantillon testé.

5. Procédé selon la revendication 1, comprenant en outre l'étape consistant à :
ajuster le pH des milieux de croissance à un pH d'approximativement 7,6 pour compenser l'ajout de FCTH.

6. Procédé selon la revendication 1, dans lequel l'étape de dilution séquentielle est effectuée en prélevant un millilitre à partir d'un flacon (302 à 312 ; 402 à 452) contenant déjà un échantillon testé mélangé et en injectant le millilitre prélevé dans un flacon séquentiel suivant (302 à 312 ; 402 à 452).

7. Procédé selon la revendication 1, dans lequel le groupe de flacons de test (302 à 312 ; 402 à 452) est incubé pendant 5 à 7 jours.

8. Procédé selon la revendication 7, dans lequel le groupe de flacons de test (302 à 312 ; 402 à 452) est incubé à 37 degrés Celsius.

9. Procédé selon la revendication 1, dans lequel le nombre de SRB présentes dans chaque flacon de test (302 à 312 ; 402 à 452) peut être estimé selon le degré de dilution auquel un noircissement visuel, qui indique la présence de sulfures de fer, est détecté visuellement dans l'échantillon en résultat des flacons de test ayant des degrés de dilution différents.

10. Procédé selon la revendication 1, comprenant en outre l'étape consistant à estimer une concentration en SRB en utilisant le procédé du nombre le plus probable (MPN).

11. Procédé selon la revendication 1, dans lequel le FCTH comprend une solution de fer mère qui est réalisée en ajoutant 1 g de FeCl₂.4H₂O dans 100 ml d'eau distillée et 0,5 ml de la solution de fer mère est ajouté à 8 millilitres des milieux de croissance et 1 ml d'échantillon testé.

12. Procédé selon la revendication 1, dans lequel les milieux de croissance comprennent une solution aqueuse qui comprend les composants suivants :
| COMPOSANT | Poids (g/1,0L) |
|---|---|
| Chlorure d'ammonium (NL₄Cl) | 1 |
| Sulfate de calcium (CaSO₄.2H₂O) | 1 |
| Sulfate de magnésium (MgSO₄.7H₂O) | 2 |
| Lactate de sodium (C₃H₅O₃Na) | 3,5 mL |
| Extrait de levure | 1 |
| Acide ascorbique | 0,1 |
| Solution de résazurine (25 mg/100 H₂O) | 4,0 mL |
| Thioglycollate de sodium (CH₂SHCOONa) | 0,1 |
| Sulfate ferreux (FeSO₄.7H₂O) | 0,5 |
| Hydrogénophosphate de potassium (K₂HPO₄) | 0,5 |
